# EUROPEAN PATENT APPLICATION

(11) **EP 4 353 710 A1**
(43) Date of publication of application: **17.04.2024**
(21) Application number: 22820618.1
(22) Date of filing: 10.06.2022
(51) Int. Cl.: C07D 209/14, A61K 31/4045, A61P 3/10, A61P 25/28, A23L 33/10, A23K 20/132

(54) **NOVEL INDOLE DERIVATIVE, AND USE THEREOF FOR TREATING DISEASES RELATED TO ADVANCED GLYCATION END PRODUCTS**

(30) Priority: 11.06.2021 KR 20210076294
(71) Applicant: Metacen Therapeutics, Suwon-si, Gyeonggi-do 16229 (KR)
(72) Inventor: SEO, Seung Yong, Incheon 22001 (KR); LEE, San Ha, Incheon 21912 (KR); LEE, Jung Eun, Siheung-si Gyeonggi-do 14923 (KR); HUR, Joon Seong, Incheon 21977 (KR); KWON, Sang Il, Incheon 21998 (KR); KIM, Sun Yeou, Seoul 06280 (KR); HONG, Seong Min, Chungju-si Chungcheongbuk-do 27356 (KR); KANG, Min Cheol, Incheon 21932 (KR); PARK, Myoung Gyu, Yongin-si Gyeonggi-do 17103 (KR); LEE, Eun Joo, Seoul 05370 (KR)
(74) Representative: Crow, Martin
(86) International application number: PCT/KR2022/008253
(87) International publication number: WO 2022/260491

(57) **Abstract**

The present invention relates to a novel indole derivative and a use thereof. The novel indole derivative according to the present invention traps methylglyoxal (MGO), which is a main precursor of advanced glycation end products, and thus can be effectively used for preventing, improving, or treating diseases related to advanced glycation end products.

## Description

### Technical Field

The present invention relates to a novel indole derivative and a use thereof for treating diseases related to advanced glycation end products. The novel indole derivative according to the present invention traps methylglyoxal (MGO), which is a main precursor of advanced glycation end products, and thus can be effectively used for preventing, improving, or treating diseases related to advanced glycation end products.

### Background Art

Diabetes mellitus is a metabolic disease in which high blood sugar levels persist for a long period of time due to insufficient secretion of insulin or insulin resistance. As elevated blood sugar levels in the body persist for a long period of time, glycation products invade the retina, kidneys, nerves, or large and small blood vessels throughout the body, causing chronic complications. Because diabetic complications are more dangerous than diabetes mellitus itself, the biggest goal in treating diabetes mellitus today is to inhibit the onset or progression of diabetic complications. Representative diabetic complications include diabetic retinopathy, diabetic cataract, diabetic nephropathy, diabetic neuropathy, diabetic heart disease, diabetic osteoporosis, diabetic atherosclerosis, and the like.

The mechanisms causing these diabetic complications are largely explained by oxidative stress caused by free radicals, non-enzymatic glycation of proteins, and osmotic stress caused by changes in the mechanism of the polyol pathway.

Oxidative stress, a cause of diabetic complications, refers to a reaction that occurs when a function of removing reactive oxygen species produced in the human body is reduced or the production of reactive oxygen species is rapidly increased due to environmental factors. It is known that in diabetic patients, oxidative stress increases due to high blood sugar, which increases insulin resistance and causes cell damage such as blood vessels, kidneys, and retina. In addition, advanced glycation end products (AGEs) produced due to oxidative stress are a major cause of diabetic complications. The glycation reaction is a non-enzymatic reaction between the aldehyde group of sugars present in blood or cell fluid and the free amino group of proteins inside and outside the cell. It refers to a series of reactions in which sugar and protein react to produce initial glycation products, and these initial glycation products are rearranged without being decomposed and then cross-linked with other proteins to produce advanced glycation products. This reaction occurs almost naturally without energy supply at the beginning of the reaction, so it occurs in food or within our bodies, and has the characteristic of being irreversible after a certain stage. Therefore, once advanced glycation end products are produced, they are not decomposed even when blood sugar levels return to normal, and accumulate in tissues during the protein survival period, abnormally changing the structure and function of tissues. In this way, glycation occurs in proteins such as basement membrane, plasma albumin, lens protein, fibrin, and collagen through non-enzymatic protein glycation reaction, and the produced advanced glycation end products abnormally change the structure and function of the tissue, causing chronic diabetic complications.

Therefore, in order to delay, prevent, or treat the onset of diabetic complications, it is important to play a role in preventing oxidative stress or to have an effect of inhibiting the production of advanced glycation end products or decomposing them.

It was recently revealed that advanced glycation end products are related to non-alcoholic steatohepatitis (NASH) disease in addition to diabetic complications. Although the pathogenesis of non-alcoholic steatohepatitis has not been fully elucidated, it is widely accepted that it is at least closely related to insulin resistance. When insulin resistance increases due to a combination of genetic factors and environmental factors related to lifestyle habits such as diet and exercise, excessive free fatty acid (FAA) is produced in the liver. Free fatty acids are converted into non-toxic triglyceride (TG) in liver cells, primarily resulting in simple steatosis (Hepatology, 2007 Jun:45(6): 1366-74; Hepatology, 2004 Jul:40(1):185-94). Later, as various oxidative stresses are added, fat peroxidation and overproduction of inflammatory cytokines cause liver cell damage and an inflammatory response, leading to non-alcoholic steatohepatitis. Non-alcoholic steatohepatitis is often accompanied by type 2 diabetes mellitus, which is another disease related to insulin resistance, and this fact suggests a relationship between advanced glycation end products, which are known to be the main causative agents of diabetic complications, and non-alcoholic steatohepatitis.

Methylglyoxal (MGO) is a main precursor of advanced glycation end products that cause complications due to diabetes mellitus. Experimental results show that MGO formation due to cellular metabolism increases at high glucose concentrations in vitro, and abnormal accumulation of MGO affects the onset of diabetic complications in various tissues and organs. In addition, there are experimental results showing that increasing MGO levels induced obesity and hyperglycemia in fruit flies. MGO is produced through several pathways, including a byproduct of glycolysis, and several mechanisms can affect MGO detoxification. MGO increased due to impaired detoxification within the organism is directly toxic to tissues and leads to the progressive development of obesity, hyperglycemia, and insulin resistance. This means that an increase in MGO can be both a result and cause of insulin resistance and hyperlipidemia, causing a potential vicious cycle (Moraru et al., 2018).

Therefore, if the production of advanced glycation end products is inhibited by trapping MGO before the production of advanced glycation end products, diabetic complications arising therefrom can be prevented. In patients with type 2 diabetes mellitus, since obesity, coronary artery disease, and the like act as risk factors, treatment of these diseases must be done simultaneously with treatment of hyperglycemia to prevent the progression of complications. However, accumulation of MGO also affects obesity. Trapping of MGO is also expected to help manage obesity in patients with type 2 diabetes mellitus.

Accordingly, the present inventors developed a novel material capable of trapping MGO, thereby completing the present invention.

### Prior Art Document

### Non-Patent Document

(Non-Patent Document 1) Yamaguchi K, et al., Hepatology. 2007 Jun;45(6): 1366-74
(Non-Patent Document 2) Feldstein AE et al., Hepatology. 2004 Jul;40(1):185-94
(Non-Patent Document 3) Moraru et al., Cell Metabolism 27, 926-934, April 3, 2018

### Detailed Description of Invention

### Technical Problem

An object of the present invention is to provide a novel compound with an excellent ability to trap methylglyoxal (MGO).

In addition, another object of the present invention is to provide a composition comprising the novel compound, wherein the composition has the effect of preventing, improving, or treating diseases related to advanced glycation end products.

### Solution to Problem

The present invention relates to a compound represented by Formula 1 below or a pharmaceutically acceptable salt thereof: 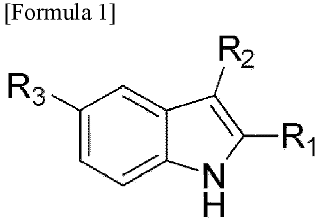 in the formula,
Ri is hydrogen, C₁-C₆alkyl, or -C₁-C₆alkyl-N(Ra)(Rb),
R₂ is hydrogen, C₁-C₆alkyl, or -C₁-C₆alkyl-C(Rc)(N(Rd)(Re)), and
R₃ is hydrogen, -OH, -O-C(=O)C₁-C₆alkyl, -O-C(=O)C₂-C₆alkenyl, or -O-C(=O)Ci-C₆alkyl-C₃-C₇cycloalkyl, wherein
Ra and Rb are each independently hydrogen or C₁-C₆alkyl,
Rc is hydrogen, -C₁-C₆alkyl-OH, -C₁-C₆alkyl-O-C(=O)C₁-C₆alkyl or -C₁-C₆alkyl-O-C(=O)NH-C₁-C₆alkyl-NH-C₁-C₆alkyl-NH₂, and
Rd and Re are each independently hydrogen or -C(=O)C₁-C₆alkyl.

In addition, Ri may be hydrogen, C₁-C₆alkyl or -C₁-C₆alkyl-NH₂.

Preferably, Ri may be hydrogen or -C₁-C₆alkyl-NH₂.

In addition, R₂ may be hydrogen or -C₁-C₆alkyl-C(Rc)(N(Rd)(Re)), wherein
Rc may be hydrogen, -C₁-C₆alkyl-OH, -C₁-C₆alkyl-O-C(=O)C₁-C₆alkyl or -Ci-C₆alkyl-O-C(=O)NH-C₁-C₆alkyl-NH-C₁-C₆alkyl-NH₂, and
Rd and Re may be each independently hydrogen or -C(=O)C₁-C₆alkyl.

Preferably, R₂ may be hydrogen,

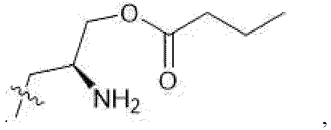

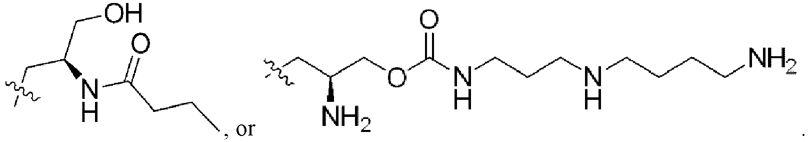

In addition, R₃ may be hydrogen, -OH, 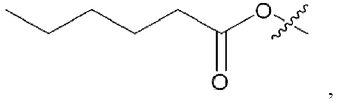 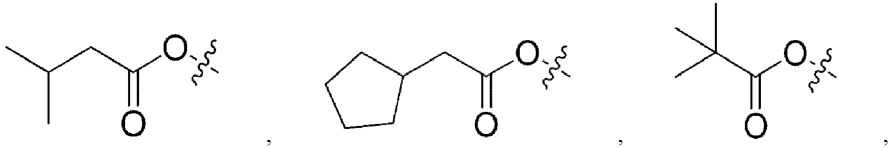 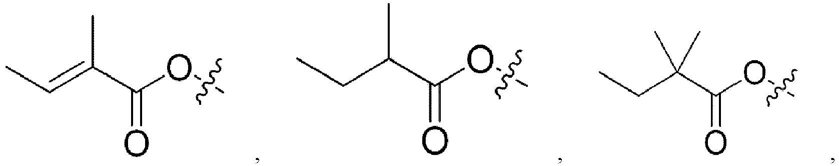 or

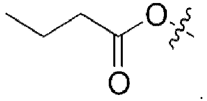

In addition, the compounds of the present invention may include the compounds shown in Table 1 below or pharmaceutically acceptable salts thereof.

**[Table 1]**

| Compound | Chemical name | Structural formula |
|---|---|---|
| 1 | 2-(aminomethyl)-1H-indol-5-yl hexanoate | 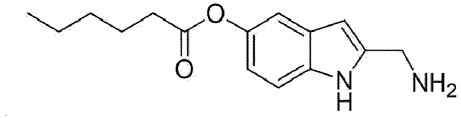 |
| 2 | 2-(aminomethyl)-1H-indol-5-yl 3-methylbutanoate | 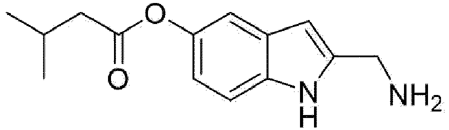 |
| 3 | 2-(aminomethyl)-1H-indol-5-yl 2-cyclopentylacetate | 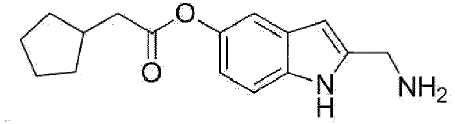 |
| 4 | 2-(aminomethyl)-1H-indol-5-yl pivalate | 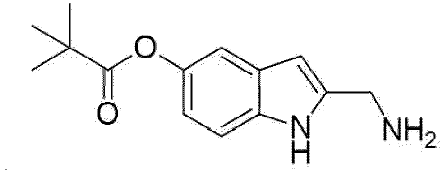 |
| 5 | 2-(aminomethyl)-1H-indol-5-yl (E)-2-methylbut-2-enoate | 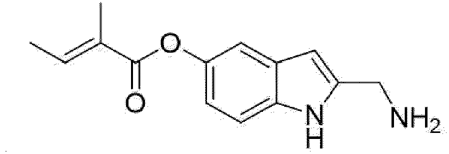 |
| 6 | 2-(aminomethyl)-1H-indol-5-yl 2-methylbutanoate | 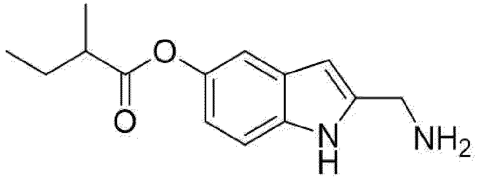 |
| 7 | 2-(aminomethyl)-1H-indol-5-yl 2,2-dimethylbutanoate | 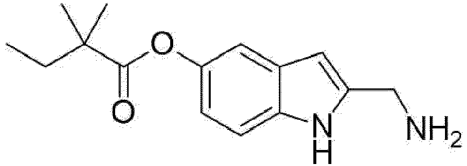 |
| 8 | (S)-2-amino-3-(5-(butyryloxy)-1H-indol-3-yl)propyl butyrate | 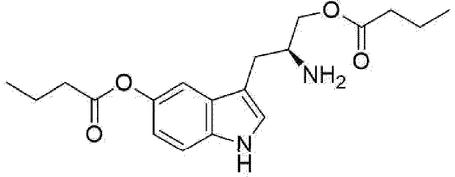 |
| 9 | (S)-3-(2-butyramido-3-hydroxypropyl)-1H-indol-5-yl butyrate | 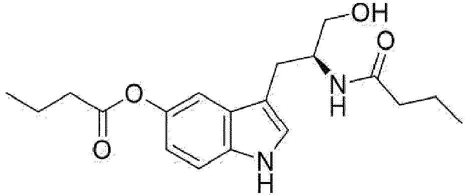 |
| 10 | (S)-2-amino-3-(5-hydroxy-1H-indol-3-yl)propyl (3-((4-aminobutyl)amino)propyl) carbamate | 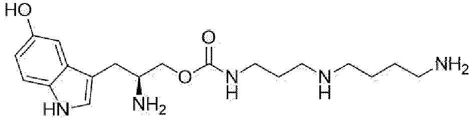 |

In addition, there is provided a pharmaceutical composition for preventing or treating diseases related to advanced glycation end products, comprising the compound or pharmaceutically acceptable salt thereof according to the present invention.

The disease related to advanced glycation end products may be selected from the group consisting of aging, diabetes mellitus, diabetic complications, hyperlipidemia, hyperglycemia, cardiovascular disease, degenerative brain disease, autism spectrum disorder, arteriosclerosis, non-alcoholic fatty liver disease, non-alcoholic steatohepatitis, skin fibrosis, pulmonary fibrosis, renal fibrosis, and cardiac fibrosis.

Preferably, the disease related to advanced glycation end products may be diabetes mellitus or diabetic complications.

The diabetes mellitus may be type 2 diabetes mellitus, and the diabetic complications may be selected from the group consisting of diabetic nephropathy, diabetic retinopathy, diabetic cataract, diabetic neuropathy, diabetic foot ulcer, diabetic cardiovascular disease, diabetic arteriosclerosis, diabetic osteoporosis, diabetic sarcopenia, and obesity.

The degenerative brain disease may be selected from the group consisting of Alzheimer's disease, Parkinson's disease, Huntington's disease, Pick's disease, Creutzfeldt-Jakob disease, Lou Gehrig's disease, spinocerebellar degeneration, Friedreich's ataxia, spinocerebellar ataxia, Machado-Joseph's disease, dystonia, progressive supranuclear palsy, cognitive dysfunction, senile dementia, Lewy body dementia, frontotemporal dementia, vascular dementia, alcoholic dementia, presenile dementia, temporal lobe epilepsy, and stroke.

In addition, there is provided a food composition for preventing or improving diseases related to advanced glycation end products, comprising the compound or pharmaceutically acceptable salt thereof according to the present invention.

The disease related to advanced glycation end products may be selected from the group consisting of aging, diabetes mellitus, diabetic complications, hyperlipidemia, hyperglycemia, cardiovascular disease, degenerative brain disease, autism spectrum disorder, arteriosclerosis, non-alcoholic fatty liver disease, non-alcoholic steatohepatitis, skin fibrosis, pulmonary fibrosis, renal fibrosis, and cardiac fibrosis.

Preferably, the disease related to advanced glycation end products may be diabetes mellitus or diabetic complications.

The diabetes mellitus may be type 2 diabetes mellitus, and the diabetic complications may be selected from the group consisting of diabetic nephropathy, diabetic retinopathy, diabetic cataract, diabetic neuropathy, diabetic foot ulcer, diabetic cardiovascular disease, diabetic arteriosclerosis, diabetic osteoporosis, diabetic sarcopenia, and obesity.

The degenerative brain disease may be selected from the group consisting of Alzheimer's disease, Parkinson's disease, Huntington's disease, Pick's disease, Creutzfeldt-Jakob disease, Lou Gehrig's disease, spinocerebellar degeneration, Friedreich's ataxia, spinocerebellar ataxia, Machado-Joseph's disease, dystonia, progressive supranuclear palsy, cognitive dysfunction, senile dementia, Lewy body dementia, frontotemporal dementia, vascular dementia, alcoholic dementia, presenile dementia, temporal lobe epilepsy, and stroke.

In addition, there is provided an animal feed composition for preventing or improving diseases related to advanced glycation end products, comprising the compound or pharmaceutically acceptable salt thereof according to the present invention.

### Effects of Invention

The present invention relates to a novel indole derivative or a pharmaceutically acceptable salt thereof. The indole derivative according to the present invention exhibits an effect of trapping methylglyoxal, a main precursor of advanced glycation end products, and an excellent effect of protecting cells. Thus, it can be effectively used as a composition for preventing, improving, or treating diseases related to advanced glycation end products.

In addition, the novel indole derivative according to the present invention can be effectively used, in particular, for preventing, improving, or treating type 2 diabetes mellitus and diabetic complications arising therefrom.

### Brief Description of Drawings

Fig. 1 shows the fluorescence intensity of the reaction product produced by the reaction between the compound of the present invention and methylglyoxal.
Fig. 2 shows the results obtained by measuring the concentration of free methylglyoxal that did not react with the compound of the present invention by high performance liquid chromatography (HPLC).
Fig. 3 shows the cell viability when MGO + _{L}-DOPA-induced SH-SY5Y cell line was treated with the compound of the present invention.
Fig. 4 shows the expression level of ΔFOSB when MGO + _{L}-DOPA-induced SH-SY5Y cell line was treated with the compound of the present invention.

### Best Mode for Carrying out the Invention

Hereinafter, with reference to the accompanying drawings, embodiments and examples of the present disclosure will be described in detail so that those of ordinary skill in the art to which the present invention belongs can easily practice the present invention. However, the present disclosure may be implemented in various forms and is not limited to the embodiments and examples described herein.

Throughout the present specification, when a certain part "includes" a certain component, it means that other components may be further included, rather than excluding other components, unless otherwise stated.

The present invention provides a compound represented by Formula 1 below or a pharmaceutically acceptable salt thereof: 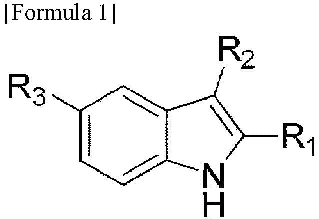 in the formula,
Ri is hydrogen, C₁-C₆alkyl, or -C₁-C₆alkyl-N(Ra)(Rb),
R₂ is hydrogen, C₁-C₆alkyl, or -C₁-C₆alkyl-C(Rc)(N(Rd)(Re)), and
R₃ is hydrogen, -OH, -O-C(=O)C₁-C₆alkyl, -O-C(=O)C₂-C₆alkenyl, or -O-C(=O)Ci-C₆alkyl-C₃-C₇cycloalkyl, wherein
Ra and Rb are each independently hydrogen or C₁-C₆alkyl,
Rc is hydrogen, -C₁-C₆alkyl-OH, -C₁-C₆alkyl-O-C(=O)C₁-C₆alkyl or -C₁-C₆alkyl-O-C(=O)NH-C₁-C₆alkyl-NH-C₁-C₆alkyl-NH₂, and
Rd and Re are each independently hydrogen or -C(=O)C₁-C₆alkyl.

In one embodiment, R₁ may be hydrogen, C₁-C₆alkyl or -C₁-C₆alkyl-NH₂.

In one embodiment, R₁ may be hydrogen or -C₁-C₆alkyl-NH₂.

In one embodiment, R₂ may be hydrogen or -C₁-C₆alkyl-C(Rc)(N(Rd)(Re)), wherein
Rc may be hydrogen, -C₁-C₆alkyl-OH, -C₁-C₆alkyl-O-C(=O)C₁-C₆alkyl or -Ci-C₆alkyl-O-C(=O)NH-C₁-C₆alkyl-NH-C₁-C₆alkyl-NH₂, and
Rd and Re may be each independently hydrogen or -C(=O)C₁-C₆alkyl.

In one embodiment, R₂ may be hydrogen,

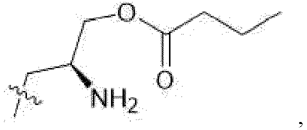

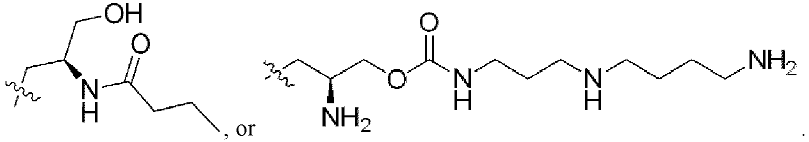

In one embodiment, R₃ may be hydrogen, -OH, 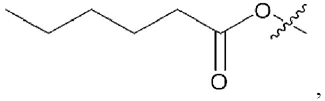 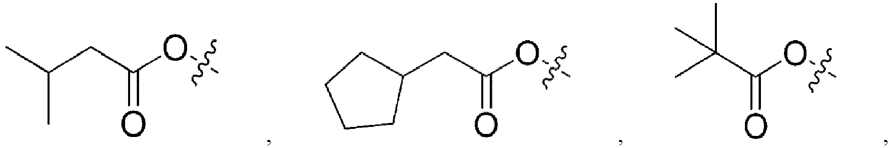 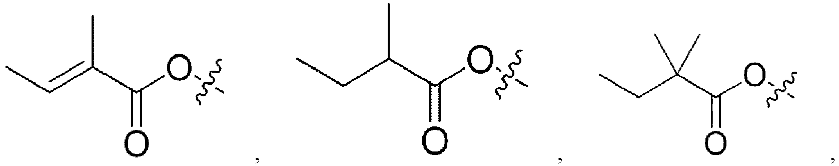 or

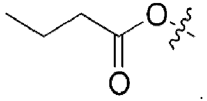

The present invention provides a composition for preventing, improving, or treating diseases related to advanced glycation end products, comprising a compound represented by Formula 1 or a pharmaceutically acceptable salt thereof.

In one embodiment, the disease related to advanced glycation end products may be selected from the group consisting of aging, diabetes mellitus, diabetic complications, hyperlipidemia, hyperglycemia, cardiovascular disease, degenerative brain disease, autism spectrum disorder, arteriosclerosis, non-alcoholic fatty liver disease, non-alcoholic steatohepatitis, skin fibrosis, pulmonary fibrosis, renal fibrosis, and cardiac fibrosis. Preferably, it is diabetes mellitus (particularly preferably type 2 diabetes mellitus) or diabetic complications.

In one embodiment, the diabetic complications may be selected from the group consisting of diabetic nephropathy, diabetic retinopathy, diabetic cataract, diabetic neuropathy, diabetic foot ulcer, diabetic cardiovascular disease, diabetic arteriosclerosis, diabetic osteoporosis, diabetic sarcopenia, and obesity.

In one embodiment, the degenerative brain disease may be selected from the group consisting of Alzheimer's disease, Parkinson's disease, Huntington's disease, Pick's disease, Creutzfeldt-Jakob disease, Lou Gehrig's disease, spinocerebellar degeneration, Friedreich's ataxia, spinocerebellar ataxia, Machado-Joseph's disease, dystonia, progressive supranuclear palsy, cognitive dysfunction, senile dementia, Lewy body dementia, frontotemporal dementia, vascular dementia, alcoholic dementia, presenile dementia, temporal lobe epilepsy, and stroke.

As used herein, the term "diabetes mellitus (DM or diabetes)" refers to a group of metabolic diseases in which high blood sugar levels persist for a long period of time. Diabetes mellitus can occur when the pancreas does not produce enough insulin or when the body's cells do not respond properly to the insulin produced. Diabetes mellitus is largely divided into type 1 diabetes mellitus, which is caused by the inability to produce enough insulin, type 2 diabetes mellitus, which is caused by insulin resistance where cells do not respond properly to insulin, and gestational diabetes.

As used herein, the term "diabetic complications" refers to symptoms that occur when diabetes mellitus persists for a long period of time. "Diabetic complications" are evaluated based on criteria different from the criteria for onset and determination of diabetes mellitus.

As used herein, the term "autism spectrum disorder (ASD)" includes a family of neurodevelopmental disorders characterized by deficits in social communication and interaction or restricted and repetitive patterns of behavior, interests or activities. Autism spectrum disorder includes autism, Asperger syndrome, pervasive developmental disorder not otherwise specified (PDD-NOS), childhood disintegrative disorder, Rett syndrome, and fragile X syndrome, but is not limited thereto.

The composition of the present invention may be a pharmaceutical composition, a food composition, or an animal feed composition.

In one embodiment, the pharmaceutical composition is suitable for medical use in humans as well as veterinary use in animals. Preferably, the animal is a mammal, which includes, without limitation, warm-blooded animals including companion animals (for example, dogs, cats, horses, etc.), and guinea pigs, mice, rats, gerbils, cows, goats, sheep, monkeys, pigs, rodents, lagomorphs, primates, and the like.

In one embodiment, the pharmaceutical composition may further comprise conventional pharmaceutically acceptable additives, such as excipients, binders, disintegrants, lubricants, solubilizers, suspending agents, preservatives, extenders, or the like.

In one embodiment, the food composition may be a health functional food, a dairy product, a fermented product, or a food additive.

In one embodiment, the food composition may further comprise various nutrients, vitamins, minerals (electrolytes), flavors such as synthetic flavors and natural flavors, coloring agents and enhancers (cheese, chocolate, etc.), pectic acid and a salt thereof, alginic acid and a salt thereof, organic acids, protective colloidal thickeners, pH adjusting agents, stabilizers, antiseptics, glycerin, alcohol, carbonation agents used in carbonated beverages, and the like.

In one embodiment, the animal feed composition may be ingested by all non-human animals, such as non-human primates, sheep, dogs, cows, horses, and the like.

In one embodiment, the animal feed composition may further comprise one or more carriers, excipients, diluents, fillers, anti-coagulants, lubricants, wetting agents, flavors, emulsifiers, preservatives, or the like.

As used herein, the term "prevention" refers to any action of inhibiting or delaying the onset of a disease by administration of a composition, and "treatment" refers to any action in which symptoms of a subject suspected of and suffering from a disease are improved or beneficially changed by administration of a composition, and "improvement" refers to any action that at least reduces a parameter related to a condition, for example, the severity of a symptom, by administration of a composition.

In accordance with the convention used in the art, " 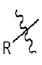 ", as used in the formulas herein, is used to indicate that a moiety or substituent "R" is attached to the backbone structure.

As used herein, the term "alkyl" is a hydrocarbon having unsubstituted or substituted primary, secondary, tertiary and/or quaternary carbon atoms, and includes a saturated aliphatic group which may be straight-chain, branched or cyclic, or a combination thereof. For example, an alkyl group may have from 1 to 20 carbon atoms (i.e., C₁-C₂₀ alkyl), from 1 to 10 carbon atoms (i.e., C₁-C₁₀ alkyl), or from 1 to 6 carbon atoms (i.e., C₁-C₆ alkyl). Unless defined otherwise, in preferred embodiments, an alkyl refers to C₁-C₆ alkyl. Examples of suitable alkyl groups may include methyl (Me, -CH₃), ethyl (Et, -CH₂CH₃), 1-propyl (n-Pr, n-propyl, - CH₂CH₂CH₃), 2-propyl (i-Pr, i-propyl, -CH(CH₃)₂), 1-butyl (n-Bu, n-butyl, - CH₂CH₂CH₂CH₃), 2-methyl-1-propyl (i-Bu, i-butyl, -CH₂CH(CH₃)₂), 2-butyl (s-Bu, s-butyl, - CH(CH₃)CH₂CH₃), 2-methyl-2-propyl (t-Bu, t-butyl, -C(CH₃)₃), 1-pentyl (n-pentyl, - CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and octyl (-(CH₂)₇CH₃), and the like, but are not limited thereto.

Moreover, as used throughout the specification, examples and claims, the term "alkyl" is intended to include both unsubstituted and substituted alkyl groups, the latter of which refers to an alkyl moiety having a substituent that replaces a hydrogen on at least one carbon of the hydrocarbon backbone, which includes a haloalkyl group such as trifluoromethyl and 2,2,2-trifluoroethyl, and the like.

As used herein, the term "C_{x-y}" or "Cₓ-C_{y}," when used in conjunction with a chemical moiety such as acyl, acyloxy, alkyl, alkenyl, alkynyl or alkoxy, is intended to include a group containing from x to y carbons in the chain. For example, a (C₁-C₆)alkyl group contains from 1 to 6 carbon atoms in the chain.

As used herein, the term "alkenyl" is a hydrocarbon that has primary, secondary, tertiary and/or quaternary carbon atoms, includes straight-chain, branched and cyclic groups, or a combination thereof, and has at least one unsaturated region, i.e., a carbon-carbon sp² double bond. For example, an alkenyl group may have from 2 to 20 carbon atoms (i.e., C₂-C₂₀ alkenyl), from 2 to 12 carbon atoms (i.e., C₂-C₁₂ alkenyl), from 2 to 10 carbon atoms (i.e., C₂-C₁₀ alkenyl), or from 2 to 6 carbon atoms (i.e., C₂-C₆ alkenyl). Examples of suitable alkenyl groups may include vinyl (-CH=CH₂), allyl (-CH₂CH=CH₂), cyclopentenyl (-C₅H₇), and 5-hexenyl (-CH₂CH₂CH₂CH₂CH=CH₂), but are not limited thereto.

As used herein, the term "alkynyl" is a hydrocarbon that has primary, secondary, tertiary and/or quaternary carbon atoms, includes straight-chain, branched and cyclic groups, or a combination thereof, and has at least one carbon-carbon sp triple bond. For example, an alkynyl group may have from 2 to 20 carbon atoms (i.e., C₂-C₂₀ alkynyl), from 2 to 12 carbon atoms (i.e., C₂-C₁₂ alkynyl), from 2 to 10 carbon atoms (i.e., C₂-C₁₀ alkynyl), or from 2 to 6 carbon atoms (i.e., C₂-C₆ alkynyl). Examples of suitable alkynyl groups may include acetylenic (-C=CH) and propargyl (-CH₂C=CH), but are not limited thereto.

As used herein, the term "cycloalkyl" refers to a monovalent or divalent, saturated or partially saturated non-aromatic ring in which the ring may be unsubstituted or substituted, monocyclic, bicyclic or polycyclic and each atom of the ring is a carbon. In addition, "cycloalkyl" can have 3 to 7 carbon atoms when it is monocyclic, 7 to 12 carbon atoms when it is bicyclic, and up to about 20 carbon atoms when it is polycyclic. A bicyclic or polycyclic ring system may be a fused ring system, a bridged ring system or a spirocyclic ring system.

As used herein, the term "heterocycloalkyl" refers to an unsubstituted or substituted, monovalent or divalent, saturated or partially saturated non-aromatic ring containing one or more heteroatoms, preferably 1 to 4 heteroatoms, more preferably 1 to 2 heteroatoms in the ring, which is monocyclic, bicyclic or polycyclic. In addition, a "heterocycloalkyl" may be a bicyclic or polycyclic ring system having two or more cyclic rings in which two or more carbons are common to two adjacent rings, wherein at least one of the rings is heterocyclic, and the other cyclic ring may be, for example, cycloalkyl, cycloalkenyl, cycloalkynyl, aryl, heteroaryl, and/or heterocycloalkyl. A bicyclic or polycyclic ring system may be a fused ring system, a bridged ring system or a spirocyclic ring system. "Heterocycloalkyl" includes, for example, piperidine, piperazine, pyrrolidine, morpholine, lactone, lactam, and the like, each of which may be unsubstituted or substituted.

As used herein, the term "halo" refers to halogen and includes chloro, fluoro, bromo, and iodo.

As used herein, the term "aryl" includes an unsubstituted or substituted, monovalent or divalent, aromatic hydrocarbon group in which each atom of the ring is carbon and the ring is monocyclic, bicyclic or polycyclic. "Aryl" may be a bicyclic or polycyclic ring system having two or more cyclic rings in which two or more carbons are common to two adjacent rings, wherein at least one of the rings is aromatic, and the other cyclic ring may be, for example, cycloalkyl, cycloalkenyl, cycloalkynyl, aryl, heteroaryl, and/or heterocycloalkyl. "Aryl" may be, for example, benzene, naphthalene, phenanthrene, anthracene, indene, indane, phenol, aniline, and the like, each of which may be unsubstituted or substituted.

As used herein, the term "Bn" refers to benzyl (-CH₂C₆H₅), and "Boc" refers to *tert-*butyloxycarbonyl (-C(=O)OC(CH₃)₃).

As used herein, the term "substituted" refers to a particular moiety of the compound of the present invention having one or more substituents. The term "substituted", for example, "substituted alkyl" or "substituted heterocycloalkyl", with regard to alkyl, heterocycloalkyl, and the like, means that one or more hydrogen atoms in the alkyl or heterocycloalkyl are each independently replaced by a non-hydrogen substituent.

As used herein, the term "pharmaceutically acceptable salt" is used herein to refer to an acid addition salt or a base addition salt that is suitable or compatible for the treatment of patients. Exemplary inorganic acids that form suitable salts may include hydrochloric acid, hydrobromic acid, sulfuric acid, and phosphoric acid, as well as metal salts such as sodium monohydrogen orthophosphate and potassium hydrogen sulfate. Exemplary organic acids that form suitable salts may include mono-, di- and tri-carboxylic acids such as glycolic acid, lactic acid, pyruvic acid, malonic acid, succinic acid, glutaric acid, fumaric acid, malic acid, tartaric acid, citric acid, ascorbic acid, maleic acid, benzoic acid, phenylacetic acid, cinnamic acid, and salicylic acid, as well as sulfonic acids such as p-toluene sulfonic acid and methanesulfonic acid. Monoacid or diacid salts may be formed, and such salts may exist in hydrated, solvated or substantially anhydrous form. In general, acid addition salts of the compound of the present invention are more soluble in water and various hydrophilic organic solvents compared to their free base forms, and generally exhibit higher melting points. Selection of appropriate salts is known to those of ordinary skill in the art.

Hereinafter, the present invention will be described in more detail through the examples, but the following examples are for illustrative purposes only and are not intended to limit the scope of the present invention.

### [Example 1]

### Preparation of 2-(aminomethyl)-1H-indol-5-yl hexanoate hydrochloride (Compound 1)

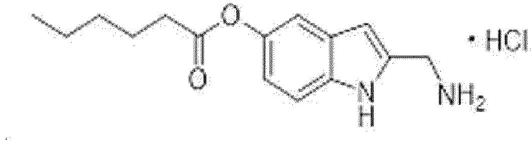

Compound 1 of the present invention was prepared according to Scheme 1 below.

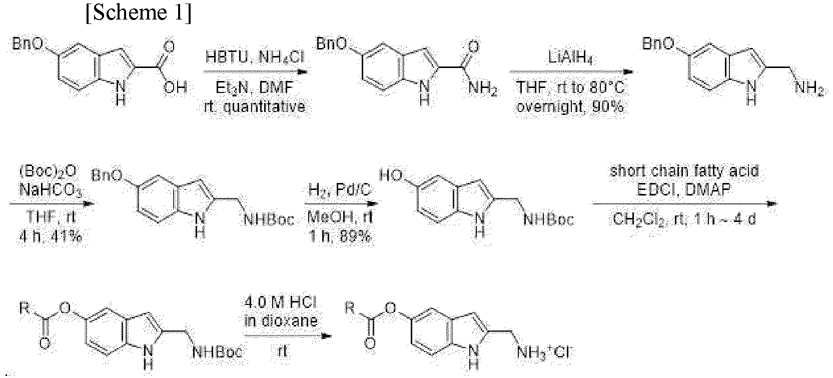

HBTU (1.40 g, 3.74 mmol) and triethylamine (1.60 mL, 11.2 mmol) were added to a solution of 5-benzyloxy-1H-indole-2-carboxylic acid (1.0 g, 3.74 mmol) in DMF (50 mL) at 0 °C. The reaction mixture was stirred overnight, and then ammonium chloride aqueous solution (20 mL) was added. The reaction was stopped by adding water to the reaction mixture, and the mixture was extracted with EtOAc. The combined organic layer was dried over brine and sodium sulfate and then concentrated under reduced pressure. The residue was purified by flash column chromatography on silica gel (CH₃OH/CH₂Cl₂/NH₄OH=1:20:2 drop) to obtain 5-(benzyloxy)-1H-indole-2-carboxamide (0.99 g, 99%); ¹H NMR (600 MHz, DMSO-d6) δ 11.44 (s, 1H), 7.95 (s, 1H), 7.47 (d, 2H, J = 7.4 Hz), 7.37 (q, 3H, J = 9.8, 8.6 Hz), 7.31 (t, 1H, J = 7.3 Hz), 7.18 (d, 1H, J = 2.0 Hz), 7.08 (m, 1H), 6.94 (m, 1H), 5.09 (s, 2H). 13C{1H} NMR (150 MHz, DMSO-d6) δ 162.9, 152.7, 137.7, 132.3, 132.0, 128.4, 127.7, 127.5, 115.0, 113.2, 103.7, 103.0, 69.7.

A solution of 5-(benzyloxy)-1H-indole-2-carboxamide (840 mg, 3.10 mmol) in THF (30 mL) was cooled to 0 °C. Lithium aluminum hydride (620 mg, 16.0 mmol) was slowly added, and the reaction mixture was allowed to warm to room temperature before being heated to reflux and stirred overnight. The reaction mixture was quenched with H₂O (10 mL) at 0 °C. The combined organic layer was washed with brine and dried over MgSO₄. The solvent was removed under reduced pressure and purified by flash column chromatography on silica gel (CH₃OH/CH₂Cl₂/NH₄OH=1:10:2 drop) to obtain (5-(benzyloxy)-1H-indol-2-yl)methanamine (790 mg, 99%); ¹H NMR (600 MHz, MeOD) δ 7.45 (m, 2H), 7.36 (t, 2H, *J=* 7.5 Hz), 7.29 (m, 2H), 7.10 (d, 1H, *J=* 2.3 Hz), 6.89 (m, 1H), 6.46 (s, 1H), 5.07 (s, 2H), 4.20 (s, 2H). ¹³C{¹H} NMR (150 MHz, MeOD) δ 154.6, 139.3, 133.6, 133.3, 130.0, 129.4, 128.7, 128.6, 114.5, 112.9, 105.0, 102.9, 71.9, 38.0.

To a solution of (5-(benzyloxy)-1H-indol-2-yl)methanamine (56, KST-42014) (740 mg, 2.90 mmol) in THF (20 mL) were added di-tert-butyl dicarbonate (0.70 mL, 3.20 mmol) and NaHCO₃ (490 mg, 5.80 mmol). The reaction mixture was stirred at room temperature for 4 hours. The reaction mixture was diluted with EtOAc, and the organic phase was washed with water and saturated NH₄Cl solution, dried over anhydrous Na₂SO₄, and then concentrated under reduced pressure. The residue was purified by flash column chromatography on silica gel (EtOAc/n-hexane=1:3) to obtain tert-butyl ((5-(benzooxy)-1H-indol-2-yl)methyl)carbamate (430 mg, 41%); ¹H NMR (600 MHz, CDCl₃) δ 8.75 (s, 1H), 7.46 (d, 2H, *J* = 7.6 Hz), 7.38 (t, 2H, *J=* 7.5 Hz), 7.31 (t, 1H, *J* = 7.3 Hz), 7.23 (d, 1H, *J* = 8.7 Hz), 7.09 (s, 1H), 6.91 (m, 1H), 6.23 (s, 1H), 5.09 (s, 2H), 5.04 (s, 1H), 4.34 (d, 2H, *J* = 5.6 Hz), 1.47 (s, 9H). ¹³C{¹H} NMR (150 MHz, CDCl₃) δ 153.5, 137.9, 131.8, 128.6, 127.9, 127.7, 113.0, 111.8, 104.1, 100.5, 71.1, 38.3, 31.1, 28.5.

To a solution of tert-butyl ((5-(benzooxy)-1H-indol-2-yl)methyl)carbamate (20.0 mg, 0.08 mmol) in CH₂Cl₂ (3.0 mL) were added hexanoic acid (10 µL, 0.09 mmol), EDCI (22 mg, 0.11 mmol), and DMAP (1.0 mg, 0.008 mmol). After stirring for 48 hours, the reaction mixture was diluted with CH₂Cl₂, washed with water and brine, dried over Na₂SO₄, and then concentrated under reduced pressure. The residue was purified by flash column chromatography on silica gel (EtOAc/n-hexane=1:2) to obtain 2-(((tert-butoxycarbonyl)amino)methyl)-1H-indol-5-yl hexanoate (26 mg, 77%); ¹H NMR (600 MHz, MeOD) δ 7.28 (d, 1H, *J* = 8.7 Hz), 7.13 (d, 1H, *J* = 2.1 Hz), 6.75 (m, 1H), 6.28 (m, 2H), 4.35 (s, 2H), 2.56 (t, 2H, *J* = 7.4 Hz), 1.75 (m, 2H), 1.46 (s, 9H), 1.41 (m, 4H), 0.95 (t, 3H, *J* = 7.1 Hz). ¹³C{¹H} NMR (150 MHz, MeOD) δ 175.1, 158.5, 145.4, 139.8, 135.9, 129.9, 116.1, 112.8, 112.1, 100.6, 80.4, 38.9, 35.1, 32.4, 28.8, 25.8, 23.4, 14.3.

2-(((tert-butoxycarbonyl)amino)methyl)-1H-indol-5-yl hexanoate (25 mg, 0.07 mmol) was dissolved in 4.0 M HCl in dioxane (2.0 mL). Then, the reaction mixture was stirred for 20 minutes and monitored by TLC. After the reaction was completed, the mixture was concentrated under reduced pressure. The obtained residue was dissolved in ethyl ether (3.0 mL) and stirred for 2 hours, and then the mixture was filtered to finally obtain Compound 1 (19 mg, 93%); ¹H NMR (600 MHz, MeOD) δ 7.39 (d, 1H, *J* = 8.7 Hz), 7.23 (d, 1H, *J* = 2.2 Hz), 6.87 (m, 1H), 6.58 (s, 1H), 4.29 (s, 2H), 2.58 (t, 2H, *J* = 7.4 Hz), 1.75 (m, 2H), 1.41 (m, 4H), 0.96 (t, 3H, *J* = 7.1 Hz). ¹³C{¹H} NMR (150 MHz, MeOD) δ 175.0, 145.9, 136.0, 132.9, 129.6, 117.8, 113.5, 112.8, 103.7, 37.6, 35.1, 32.4, 25.8, 23.4, 14.3.

### [Example 2]

### Preparation of 2-(aminomethyl)-1H-indol-5-yl 3-methylbutanoate hydrochloride

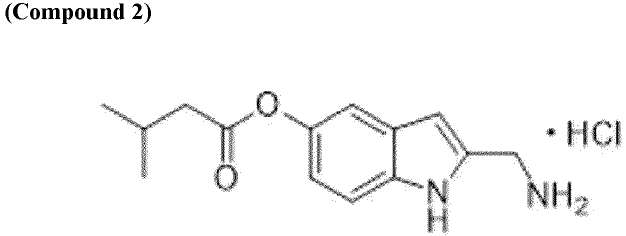

To a solution of tert-butyl ((5-hydroxy-1H-indol-2-yl)methyl)carbamate (20.0 mg, 0.08 mmol) in CH₂Cl₂ (3.0 mL) were added isovaleric acid (10 µL, 0.09 mmol), EDCI (22 mg, 0.11 mmol), and DMAP (1.1 mg, 0.008 mmol). After stirring for 2 hours, the reaction mixture was diluted with CH₂Cl₂, washed with water and brine, dried over Na₂SO₄, and then concentrated under reduced pressure. The residue was purified by flash column chromatography on silica gel (EtOAc/n-hexane=1:2) to obtain 2-(((tert-butoxycarbonyl)amino)methyl)-1H-indol-5-yl 3-methylbutanoate (23 mg, 85%); ¹H NMR (600 MHz, MeOD) δ 7.29 (d, 1H, *J* = 8.7 Hz), 7.12 (d, 1H, *J* = 2.1 Hz), 6.75 (m, 1H), 6.29 (m, 1H), 4.35 (s, 2H), 2.44 (d, 2H, *J* = 7.2 Hz), 2.21 (m, 1H), 1.46 (s, 9H), 1.07 (s, 3H), 1.06 (s, 3H). ¹³C{¹H} NMR (150 MHz, MeOD) δ 174.4, 158.5, 145.4, 139.8, 135.9, 129.9, 116.1, 112.8, 112.2, 100.6, 80.4, 44.2, 38.9, 28.8, 27.1, 22.7.

2-(((tert-butoxycarbonyl)amino)methyl)-1H-indol-5-yl 3-methylbutanoate (21 mg, 0.06 mmol) was dissolved in 4.0 M HCl in dioxane (2.0 mL). Then, the reaction mixture was stirred for 10 minutes and monitored by TLC. After the reaction was completed, the mixture was concentrated under reduced pressure. The obtained residue was dissolved in ethyl ether (3.0 mL) and stirred for 2 hours, and then the mixture was filtered to finally obtain Compound 2 (17 mg, 97%); ¹H NMR (600 MHz, MeOD) δ 7.40 (d, 1H*, J* = 8.7 Hz), 7.22 (d, 1H, *J* = 2.1 Hz), 6.87 (m, 1H), 6.59 (s, 1H), 4.29 (s, 2H), 2.46 (d, 2H, *J* = 7.2 Hz), 2.26 - 2.17 (m, 1H), 1.08 (s, 3H), 1.07 (s, 3H). ¹³C{¹H} NMR (150 MHz, MeOD) δ 174.2, 145.9, 136.0, 132.9, 129.6, 117.8, 113.5, 112.8, 103.7, 44.2, 37.6, 27.1, 22.7.

### [Example 3]

### Preparation of 2-(aminomethyl)-1H-indol-5-yl 2-cyclopentylacetate hydrochloride

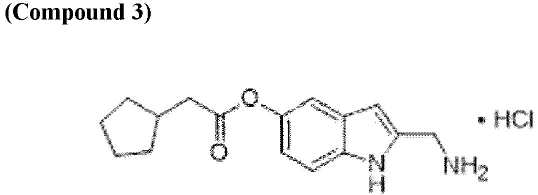

To a solution of tert-butyl ((5-hydroxy-1H-indol-2-yl)methyl)carbamate (20.0 mg, 0.08 mmol) in CH₂Cl₂ (3.0 mL) were added 2-cyclopentyl acetic acid (10 µL, 0.09 mmol), EDCI (22 mg, 0.11 mmol), and DMAP (1.1 mg, 0.008 mmol). After stirring for 3 hours, the reaction mixture was diluted with CH₂Cl₂, washed with water and brine, dried over Na₂SO₄, and then concentrated under reduced pressure. The residue was purified by flash column chromatography on silica gel (EtOAc/n-hexane=1:2) to obtain 2-(((tert-butoxycarbonyl)amino)methyl)-1H-indol-5-yl 2-cyclopentylacetate (30 mg, 98%); ¹H NMR (600 MHz, MeOD) δ 7.28 (d, 1H, *J* = 8.6 Hz), 7.12 (d, 1H, *J* = 2.1 Hz), 6.75 (m, 1H), 6.29 (m, 1H), 4.35 (s, 2H), 2.56 (d, 2H, *J=* 7.5 Hz), 2.35 (m, 1H), 1.94 (m, 2H), 1.71 (m, 2H), 1.63 (m, 2H), 1.46 (s, 9H), 1.30 (m, 2H). ¹³C{¹H} NMR (150 MHz, MeOD) δ 174.7, 158.5, 145.4, 139.8, 135.9, 129.9, 116.1, 112.8, 112.1, 100.6, 80.4, 41.3, 38.9, 38.0, 37.8, 33.4, 26.0.

2-(((tert-butoxycarbonyl)amino)methyl)-1H-indol-5-yl 2-cyclopentylacetate (26 mg, 0.07 mmol) was dissolved in 4.0 M HCl in dioxane (2.0 mL). Then, the reaction mixture was stirred for 15 minutes and monitored by TLC. After the reaction was completed, the mixture was concentrated under reduced pressure. The obtained residue was dissolved in ethyl ether (3.0 mL) and stirred for 2 hours, and then the mixture was filtered to finally obtain Compound 3 (19 mg, 90%); ¹H NMR (600 MHz, MeOD) δ 7.39 (d, 1H, *J* = 8.7 Hz), 7.22 (d, 1H*, J* = 2.1 Hz), 6.87 (m, 1H), 6.59 (s, 1H), 4.29 (s, 2H), 2.58 (d, 2H, *J* = 7.5 Hz), 2.37 (m, 1H), 1.94 (m, 2H), 1.73 (m, 2H), 1.64 (m, 2H), 1.34 (m, 2H). ¹³C{¹H} NMR (150 MHz, MeOD) δ 174.5, 145.9, 136.0, 132.9, 129.6, 117.8, 113.5, 112.8, 103.7, 41.2, 38.0, 37.5, 33.4, 26.0.

### [Example 4]

### Preparation of 2-(aminomethyl)-1H-indol-5-yl pivalate hydrochloride (Compound 4)

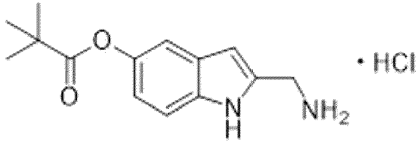

To a solution of tert-butyl ((5-hydroxy-1H-indol-2-yl)methyl)carbamate (20.0 mg, 0.08 mmol) in CH₂Cl₂ (3.0 mL) were added pivalic acid (9.2 mg, 0.09 mmol), EDCI (22 mg, 0.11 mmol), and DMAP (1.2 mg, 0.008 mmol). After stirring for 72 hours, the reaction mixture was diluted with CH₂Cl₂, washed with water and brine, dried over Na₂SO₄, and then concentrated under reduced pressure. The residue was purified by flash column chromatography on silica gel (EtOAc/n-hexane=1:2) to obtain 2-(((tert-butoxycarbonyl)amino)methyl)-1H-indol-5-yl pivalate (17 mg, 64%); ¹H NMR (600 MHz, MeOD) δ 7.29 (d, 1H, *J* = 8.6 Hz), 7.10 (d, 1H, *J* = 2.1 Hz), 6.72 (m, 1H), 6.28 (m, 1H), 4.36 (s, 2H), 1.47 (s, 9H), 1.36 (s, 9H). ¹³C{¹H} NMR (150 MHz, MeOD) δ 179.8, 158.5, 145.7, 139.8, 135.9, 129.9, 115.9, 112.7, 112.2, 100.6, 80.4, 40.0, 38.9, 28.8, 27.6, 27.6.

2-(((tert-butoxycarbonyl)amino)methyl)-1H-indol-5-yl pivalate (17 mg, 0.05 mmol) was dissolved in 4.0 M HCl in dioxane (2.0 mL). Then, the reaction mixture was stirred for 20 minutes and monitored by TLC. After the reaction was completed, the mixture was concentrated under reduced pressure. The obtained residue was dissolved in ethyl ether (3.0 mL) and stirred for 2 hours, and then the mixture was filtered to finally obtain Compound 4 (13 mg, 97%); ¹H NMR (600 MHz, MeOD) δ 7.40 (d, 1H, *J=* 8.7 Hz), 7.20 (d, 1H, *J=* 2.1 Hz), 6.84 (m, 1H), 6.59 (s, 1H), 4.29 (s, 2H), 1.37 (s, 9H). ¹³C{¹H} NMR (150 MHz, MeOD) δ 179.7, 146.2, 136.0, 132.9, 129.6, 117.6, 113.3, 112.8, 103.7, 40.0, 37.5, 27.6.

### [Example 5]

### Preparation of 2-(aminomethyl)-1H-indol-5-yl (E)-2-methylbut-2-enoate hydrochloride

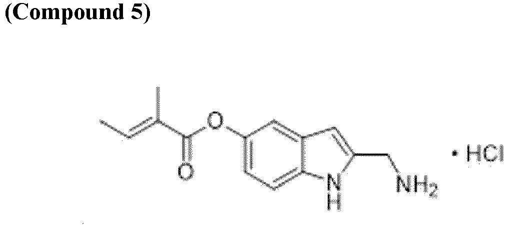

To a solution of tert-butyl ((5-hydroxy-1H-indol-2-yl)methyl)carbamate (20.0 mg, 0.08 mmol) in CH₂Cl₂ (3.0 mL) were added tiglic acid (9.2 mg, 0.09 mmol), EDCI (22 mg, 0.11 mmol), and DMAP (1.2 mg, 0.008 mmol). After stirring for 5 hours, the reaction mixture was diluted with CH₂Cl₂, washed with water and brine, dried over Na₂SO₄, and then concentrated under reduced pressure. The residue was purified by flash column chromatography on silica gel (EtOAc/n-hexane=1:2) to obtain 2-(((tert-butoxycarbonyl)amino)methyl)-1H-indol-5-yl (E)-2-methylbut-2-enoate (20 mg, 77%); ¹H NMR (600 MHz, MeOD) δ 7.29 (d, 1H, *J* = 8.7 Hz), 7.14 (d, 1H, *J* = 2.0 Hz), 7.11 (m, 2H), 6.77 (m, 1H), 6.28 (s, 1H), 4.36 (s, 2H), 1.95 (s, 3H), 1.90 (m, 3H), 1.47 (s, 9H). ¹³C{¹H} NMR (150 MHz, MeOD) δ 169.2, 158.6, 145.7, 140.2, 139.8, 135.8, 130.0, 129.6, 116.2, 112.9, 112.2, 100.6, 80.4, 38.9, 28.8, 14.6, 12.3.

2-(((tert-butoxycarbonyl)amino)methyl)-1H-indol-5-yl (E)-2-methylbut-2-enoate (20 mg, 0.06 mmol) was dissolved in 4.0 M HCl in dioxane (2.0 mL). Then, the reaction mixture was stirred for 30 minutes and monitored by TLC. After the reaction was completed, the mixture was concentrated under reduced pressure. The obtained residue was dissolved in ethyl ether (3.0 mL) and stirred for 2 hours, and then the mixture was filtered to finally obtain Compound 5 (15 mg, 88%); ¹H NMR (600 MHz, MeOD) δ 7.40 (d, 1H, *J* = 8.7 Hz), 7.25 (d, 1H, *J* = 2.1 Hz), 7.12 (m, 1H), 6.89 (m, 1H), 6.59 (s, 1H), 4.30 (s, 2H), 1.96 (s, 3H), 1.88 (m, 3H). ¹³C{¹H} NMR (150 MHz, MeOD) δ 169.0, 146.2, 140.4, 136.0, 132.8, 129.6, 129.5, 117.9, 113.6, 112.8, 103.7, 37.6, 14.6, 12.3.

### [Example 6]

### Preparation of 2-(aminomethyl)-1H-indol-5-yl 2-methylbutanoate hydrochloride

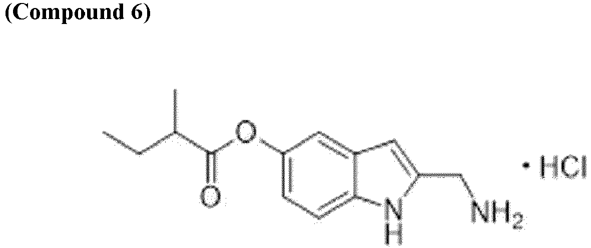

To a solution of tert-butyl ((5-hydroxy-1H-indol-2-yl)methyl)carbamate (20.0 mg, 0.08 mmol) in CH₂Cl₂ (3.0 mL) were added DL-2-methylbutanoic acid (10 µL, 0.09 mmol), EDCI (22 mg, 0.11 mmol), and DMAP (1.0 mg, 0.008 mmol). After stirring for 24 hours, the reaction mixture was diluted with CH₂Cl₂, washed with water and brine, dried over Na₂SO₄, and then concentrated under reduced pressure. The residue was purified by flash column chromatography on silica gel (EtOAc/n-hexane=1:2) to obtain 2-(((tert-butoxycarbonyl)amino)methyl)-1H-indol-5-yl 2-methylbutanoate (20 mg, 77%); ¹H NMR (600 MHz, MeOD) δ 7.29 (d, 1H, *J* = 8.6 Hz), 7.12 (d, 1H, *J* = 2.0 Hz), 6.74 (m, 1H), 6.28 (s, 1H), 4.36 (s, 2H), 2.64 (q, 1H, *J* = 7.0 Hz), 1.81 (m, 1H), 1.65 (m, 1H), 1.47 (s, 9H), 1.28 (d, 3H, *J* = 7.0 Hz), 1.05 (t, 3H, *J* = 7.5 Hz). ¹³C{¹H} NMR (150 MHz, MeOD) δ 178.0, 158.5, 145.4, 139.8, 135.9, 129.9, 116.0, 112.8, 112.2, 100.6, 80.4, 42.4, 38.9, 28.8, 28.0, 17.1, 12.0.

2-(((tert-butoxycarbonyl)amino)methyl)-1H-indol-5-yl 2-methylbutanoate (20 mg, 0.06 mmol) was dissolved in 4.0 M HCl in dioxane (2.0 mL). Then, the reaction mixture was stirred for 30 minutes and monitored by TLC. After the reaction was completed, the mixture was concentrated under reduced pressure. The obtained residue was dissolved in ethyl ether (3.0 mL) and stirred for 2 hours, and then the mixture was filtered to finally obtain Compound 6 (15 mg, 88%); ¹H NMR (600 MHz, MeOD) δ 7.40 (d, 1H, *J* = 8.7 Hz), 7.22 (d, 1H*, J* = 2.1 Hz), 6.86 (m, 1H), 6.59 (s, 1H), 4.29 (s, 2H), 2.66 (m, 1H), 1.82 (m, 1H), 1.65 (m, 1H), 1.29 (d, 3H, *J* = 7.0 Hz), 1.05 (t, 3H, *J* = 7.5 Hz). ¹³C{¹H} NMR (150 MHz, MeOD) δ 177.9, 145.9, 136.0, 132.9, 129.6, 117.7, 113.4, 112.8, 103.7, 42.4, 37.6, 28.0, 17.1, 12.0.

### [Example 7]

### Preparation of 2-(aminomethyl)-1H-indol-5-yl 2,2-dimethylbutanoate hydrochloride

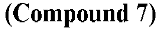

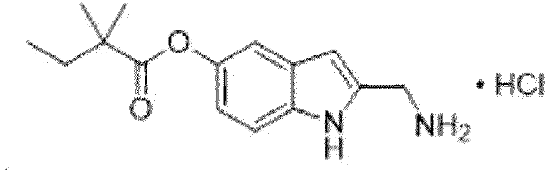

To a solution of tert-butyl ((5-hydroxy-1H-indol-2-yl)methyl)carbamate (20.0 mg, 0.08 mmol) in CH₂Cl₂ (3.0 mL) were added 2,2-dimethyl butyric acid (10 µL, 0.09 mmol), EDCI (22 mg, 0.11 mmol), and DMAP (1.0 mg, 0.008 mmol). After stirring for 96 hours, the reaction mixture was diluted with CH₂Cl₂, washed with water and brine, dried over Na₂SO₄, and then concentrated under reduced pressure. The residue was purified by flash column chromatography on silica gel (EtOAc/n-hexane=1:2) to obtain 2-(((tert-butoxycarbonyl)amino)methyl)-1H-indol-5-yl 2,2-dimethylbutanoate (12 mg, 43%); 1HNMR (600 MHz, MeOD) δ 7.29 (d, 1H, J = 8.6 Hz), 7.09 (d, 1H, J = 2.1 Hz), 6.71 (m, 1H), 6.28 (s, 1H), 4.36 (s, 2H), 1.75 (q, 2H, J = 7.5 Hz), 1.47 (s, 9H), 1.31 (s, 6H), 1.01 (t, 3H, J = 7.5 Hz). ¹³C{¹H} NMR (150 MHz, MeOD) δ 179.3, 158.6, 145.6, 139.9, 135.9, 130.0, 116.0, 112.7, 112.2, 100.6, 80.4, 44.0, 38.9, 34.5, 28.8, 25.2, 9.7.

2-(((tert-butoxycarbonyl)amino)methyl)-1H-indol-5-yl 2,2-dimethylbutanoate (12 mg, 0.03 mmol) was dissolved in 4.0 M HCl in dioxane (1.0 mL). Then, the reaction mixture was stirred for 30 minutes and monitored by TLC. After the reaction was completed, the mixture was concentrated under reduced pressure. The obtained residue was dissolved in ethyl ether (2.0 mL) and stirred for 2 hours, and then the mixture was filtered to finally obtain Compound 7 (9.0 mg, 91%); 1H NMR (600 MHz, MeOD) δ 7.40 (d, 1H, J = 8.7 Hz), 7.19 (d, 1H, J = 2.1 Hz), 6.83 (m, 1H), 6.59 (s, 1H), 4.29 (s, 2H), 1.76 (q, 2H, J = 7.5 Hz), 1.32 (s, 6H), 1.01 (t, 3H, J = 7.5 Hz). 13C{1H} NMR (150 MHz, MeOD) δ 179.1, 146.1, 136.0, 133.0, 129.7, 117.7, 113.4, 112.8, 103.6, 44.0, 37.6, 34.5, 25.2, 9.7.

### [Example 8]

### Preparation of (S)-2-amino-3-(5-(butyryloxy)-1H-indol-3-yl)propyl butyrate (Compound 8)

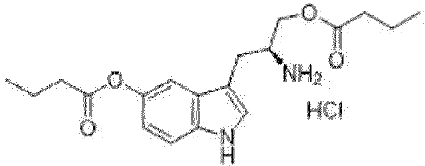

Compound 8 of the present invention was prepared according to Scheme 2 below.

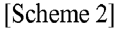

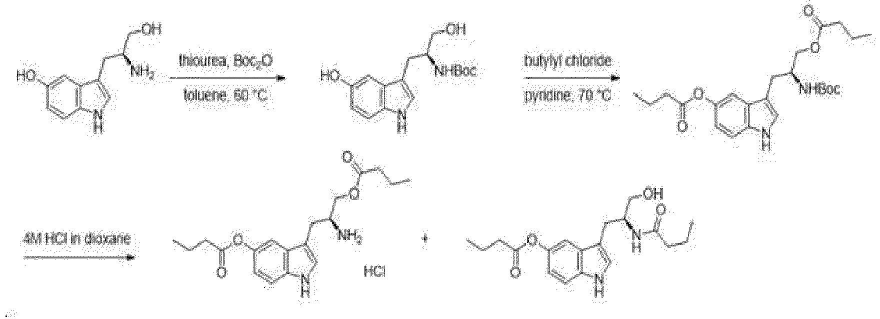

To a solution of (S)-3-(2-amino-3-hydroxypropyl)-1H-indol-5-ol (100 mg, 0.48 mmol) and Boc₂O (105 mg, 0.48 mmol) in toluene (2.5 mL) was added thiourea (4 mg, 0.05 mmol). After stirring at 60 °C for 30 minutes, the reaction mixture was filtered through a Celite pad and concentrated under reduced pressure. The residue was purified by flash column chromatography on silica gel (EtOAc/Hexane/MeOH=1:1:0.1) to obtain tert-butyl (S)-(1-hydroxy-3-(5-hydroxy-1H-indol-3-yl)propan-2-yl)carbamate (148 mg, 92%, brown caramel).

To a solution of tert-butyl (S)-(1-hydroxy-3-(5-hydroxy-1H-indol-3-yl)propan-2-yl)carbamate (55 mg, 0.18 mmol) in pyridine (1 mL) was added butyryl chloride (0.05 mL, 0.45 mmol). After stirring at 70 °C for 3 hours, the reaction mixture was quenched with aqueous NH₄Cl and extracted with EtOAc. The combined organic layer was washed with brine, dried over MgSO₄, and then concentrated under reduced pressure. The residue was purified by flash column chromatography on silica gel (EtOAc/Hexane=1:3) to obtain (S)-2-((tert-butoxycarbonyl)amino)-3-(5-(butyryloxy)-1H-indol-3-yl)propyl butyrate (50 mg, 63%, clear oil).

To (S)-2-((tert-butoxycarbonyl)amino)-3-(5-(butyryloxy)-1H-indol-3-yl)propyl butyrate (26 mg, 0.06 mmol) at 0 °C was added a 4 M solution of HCl in 1,4-dioxane (1 mL). After stirring at the same temperature for 1 hour, the mixture was directly concentrated under reduced pressure to obtain Compound 8. Compound 8 was redissolved in CH₂Cl₂ and left for 2 days to partially be converted to Compound 9. The mixture of Compounds 8 and 9 was purified by flash column chromatography on silica gel (CH₂Cl₂/MeOH=14:1) to finally obtain Compound 8 (10 mg, 45%, white solid); ¹H NMR (600 MHz, DMSO) δ 10.85 (d, J = 2.5 Hz, 1H), 7.56 (d, J = 8.1 Hz, 1H), 7.29 (d, J = 8.7 Hz, 1H), 7.25 (d, J = 2.2 Hz, 1H), 7.13 (d, J = 2.3 Hz, 1H), 6.76 (dd, J = 8.6, 2.3 Hz, 1H), 4.69 (t, J = 5.6 Hz, 1H), 3.91 (ddd, J = 7.9, 6.8, 3.9 Hz, 1H), 3.36 (dt, J = 10.7, 5.3 Hz, 1H), 3.29 (m, 1H), 2.86 (dd, J = 14.5, 5.9 Hz, 1H), 2.67 (dd, J = 14.5, 7.5 Hz, 1H), 2.54 (t, J = 7.3 Hz, 2H), 1.99 (td, J = 7.3, 1.7 Hz, 2H), 1.67 (h, J = 7.3 Hz, 2H), 1.44 (h, J = 7.4 Hz, 2H), 0.98 (t, J = 7.4 Hz, 3H), 0.77 (t, J = 7.4 Hz, 3H).

### [Example 9]

### Preparation of (S)-3-(2-butyramido-3-hydroxypropyl)-1H-indol-5-yl butyrate

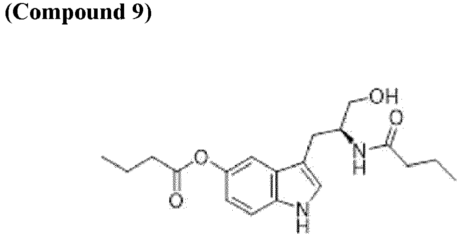

Compound 9 of the present invention was prepared according to Scheme 2 of Example 8 above and obtained in the same manner as in Compound 8 above.

To (S)-2-((tert-butoxycarbonyl)amino)-3-(5-(butyryloxy)-1H-indol-3-yl)propyl butyrate (26 mg, 0.06 mmol) at 0 °C was added a 4 M solution of HCl in 1,4-dioxane (1 mL). After stirring at the same temperature for 1 hour, the mixture was directly concentrated under reduced pressure to obtain Compound 8. Compound 8 was redissolved in CH₂Cl₂ and left for 2 days to partially be converted to Compound 9. The mixture of Compounds 8 and 9 was purified by flash column chromatography on silica gel (CH₂Cl₂/MeOH=14:1) to finally obtain Compound 9 (7 mg, 35%, white solid); ¹H NMR (600 MHz, DMSO) δ 11.13 (d, J = 2.5 Hz, 1H), 8.11 (s, 3H), 7.36 (d, J = 8.7 Hz, 1H), 7.30 (dd, J = 8.2, 2.3 Hz, 2H), 6.82 (dd, J = 8.7, 2.2 Hz, 1H), 4.13 (dd, J = 12.0, 3.5 Hz, 1H), 4.04 (dd, J = 12.0, 6.3 Hz, 1H), 3.60 (bs, 1H), 2.99 (ddd, J = 23.1, 14.6, 7.2 Hz, 2H), 2.54 (t, J = 7.3 Hz, 2H), 2.33 (td, J = 7.3, 1.9 Hz, 2H), 1.67 (h, J = 7.4 Hz, 2H), 1.55 (h, J = 7.4 Hz, 2H), 0.98 (t, J = 7.4 Hz, 3H), 0.88 (t, J = 7.4 Hz, 3H).

### [Example 10]

### Preparation of (S)-2-amino-3-(5-hydroxy-1H-indol-3-yl)propyl (3-((4-aminobutyl)amino)propyl)carbamate (Compound 10)

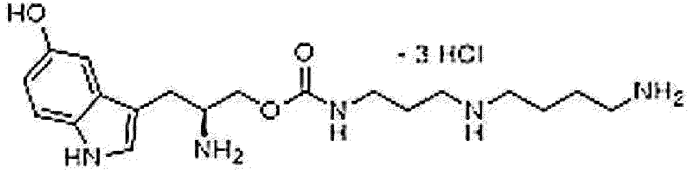

Compound 10 of the present invention was prepared according to Scheme 3 below.

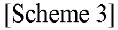

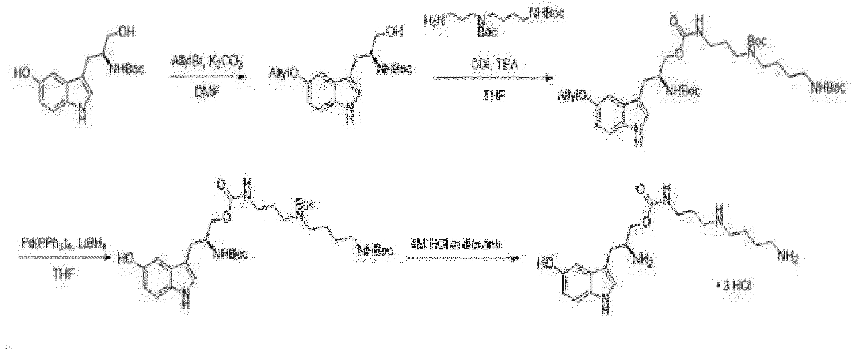

To a solution of tert-butyl (S)-(1-hydroxy-3-(5-hydroxy-1H-indol-3-yl)propan-2-yl)carbamate (260 mg, 0.85 mmol) in DMF (4 mL) at room temperature were added K₂CO₃ (176 mg, 1.27 mmol) and alkyl bromide (0.1 mL, 1.02 mmol). After stirring at the same temperature for 3 days, the reaction mixture was quenched with water and extracted with Et₂O. The combined organic layer was washed with brine, dried over MgSO₄, and then concentrated under reduced pressure. The residue was purified by flash column chromatography on silica gel (EtOAc/Hexane=2:3) to obtain tert-butyl (S)-(1-(5-(allyloxy)-1H-indol-3-yl)-3-hydroxypropan-2-yl)carbamate (257 mg, 87%, brown caramel); ¹H NMR (600 MHz, CDCl₃) δ 8.01 (s, 1H), 7.23 (s, 1H), 7.10 (d, J = 2.4 Hz, 1H), 7.02 (s, 1H), 6.88 (dd, J = 8.8, 2.4 Hz, 1H), 6.09 (ddt, J = 17.3, 10.6, 5.4 Hz, 1H), 5.43 (dq, J = 17.1, 1.6 Hz, 1H), 5.26 (dq, J = 10.4, 1.4 Hz, 1H), 4.57 (dt, J = 5.4, 1.5 Hz, 2H), 3.94 (p, J = 6.4 Hz, 1H), 3.68 (dd, J = 10.9, 3.9 Hz, 1H), 3.59 (dd, J = 11.0, 5.5 Hz, 1H), 2.93 (m, 2H), 1.39 (s, 9H).

To an anhydrous THF solution (1 mL) of tert-butyl (S)-(1-(5-(allyloxy)-1H-indol-3-yl)-3-hydroxypropan-2-yl)carbamate (41 mg, 0.12 mmol) at 0 °C was added carbonyldiimidazole (21 mg, 0.13 mmol). The mixture was activated at room temperature for 1 hour, and N1,N5-Bis-Boc-spermidine (32 mg, 0.09 mmol) and triethylamine (0.05 mL, 0.36 mmol) were added successively. After stirring overnight, the reaction mixture was quenched with 1N HCl and extracted with EtOAc. The combined organic layer was washed with aqueous NaHCO₃, dried over MgSO₄, and then concentrated under reduced pressure. The residue was purified by flash column chromatography on silica gel (Acetone/Hexane=1:2) to obtain tert-butyl (S)-(6-((5-(allyloxy)-1H-indol-3-yl)methyl)-2,2-dimethyl-4,9-dioxo-3,8-dioxa-5,10-diazatridecan-13-yl)(4-((tert-butoxycarbonyl)amino)butyl)carbamate (39 mg, 45%, colorless oil); ¹H NMR (600 MHz, MeOD) δ 7.21 (d, J = 8.7 Hz, 1H), 7.13 (s, 1H), 7.05 (s, 1H), 6.78 (d, J = 8.7 Hz, 1H), 6.12 (ddt, J = 17.3, 10.5, 5.3 Hz, 1H), 5.43 (d, J = 17.2 Hz, 1H), 5.24 (ddd, J = 10.5, 3.0, 1.5 Hz, 1H), 4.57 (d, J = 4.0 Hz, 2H), 4.02 (m, 3H), 3.24 (s, 2H), 3.19 (t, J = 7.3 Hz, 2H), 3.10 (t, J = 6.6 Hz, 2H), 3.03 (t, J = 6.8 Hz, 2H), 2.88 (m, 2H), 1.73 (bs, 2H), 1.54 (m, 2H), 1.43 (m, 2H), 1.42 (s, 18H), 1.40 (s, 9H).

To an anhydrous THF solution (1 mL) of tert-butyl (S)-(6-((5-(allyloxy)-1H-indol-3-yl)methyl)-2,2-dimethyl-4,9-dioxo-3,8-dioxa-5,10-diazatridecan-13-yl)(4-((tert-butoxycarbonyl)amino)butyl)carbamate (36 mg, 0.05 mmol) at room temperature were added Pd(PPh₃)₄ (6 mg, 0.01 mmol) and LiBH₄ (5 mg, 0.25 mmol). After stirring overnight, the reaction mixture was quenched with 1N HCl and extracted with EtOAc. The combined organic layer was dried over MgSO₄ and then concentrated under reduced pressure. The residue was purified by flash column chromatography on silica gel (EtOAc/Hexane=3:2) to obtain tert-butyl (S)-(4-((tert-butoxycarbonyl)amino)butyl)(6-((5-hydroxy-1H-indol-3-yl)methyl)-2,2-dimethyl-4,9-dioxo-3,8-dioxa-5,10-diazatridecan-13-yl)carbamate (12 mg, 35%, colorless oil); ¹H NMR (600 MHz, Methanol-d4) δ 7.15 (d, J = 8.7 Hz, 1H), 7.01 (s, 1H), 6.97 (s, 1H), 6.66 (dd, J = 8.6, 2.4 Hz, 1H), 4.03 (m, 3H), 3.20 (m, 4H), 3.10 (t, J = 6.6 Hz, 2H), 3.03 (t, J = 6.7 Hz, 2H), 2.86 (bs, 2H), 1.72 (bs, 2H), 1.53 (bs, 2H), 1.43 (m, 29H).

To tert-butyl (S)-(4-((tert-butoxycarbonyl)amino)butyl)(6-((5-hydroxy-1H-indol-3-yl)methyl)-2,2-dimethyl-4,9-dioxo-3,8-dioxa-5,10-diazatridecan-13-yl)carbamate (12 mg, 0.02 mmol) at 0 °C was added a 4 M solution of HCl in 1,4-dioxane (1 mL). After stirring at the same temperature for 1 hour, the mixture was directly concentrated under reduced pressure to finally obtain Compound 10 (11 mg, 100%, white powder); ¹H NMR (600 MHz, Methanol-d4) δ 7.20 (d, J = 8.6 Hz, 1H), 7.14 (s, 1H), 6.94 (s, 1H), 6.71 (dd, J = 8.7, 2.0 Hz, 1H), 4.27 (dd, J = 12.0, 3.4 Hz, 1H), 4.10 (dd, J = 12.0, 6.3 Hz, 1H), 3.73 (m, 1H), 3.64 (m, 1H), 3.58 (m, 1H), 3.26 (m, 2H), 3.03 (m, 8H), 1.92 (m, 2H), 1.77 (m, 4H).

### [Experimental Example 1]

### Analysis of methylglyoxal (MGO) trapping ability (1) : analysis of fluorescence intensity

Methylglyoxal (MGO) is a main precursor of advanced glycation end products that cause complications due to diabetes mellitus. Successful trapping of methylglyoxal (MGO) can inhibit the production of advanced glycation end products. Therefore, the objective of this experimental example was to confirm whether the compound of the present invention has the ability to trap methylglyoxal (MGO), a precursor before the production of advanced glycation end products.

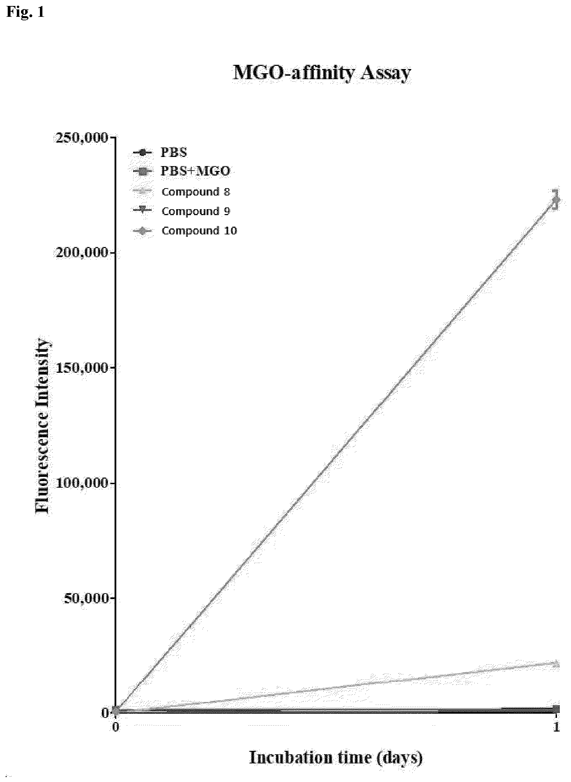

A mixture was prepared by adding methylglyoxal (MGO) to phosphate buffered saline (PBS, Cat no. 10010023, pH 7.4). The mixture (MGO+PBS) was treated with the compound of the above example at a concentration of 1.0 mM and then cultured at 37 °C for 1 day. The fluorescence intensity of the reaction product was measured at an excitation wavelength of 355 nm and an emission wavelength of 460 nm using a VICTOR^{™}X3 multilabel plate reader. The fluorescence intensity obtained for each compound is shown in Table 2 below and Fig. 1.

The reaction product produced when the compound reacts with methylglyoxal exhibits fluorescence. The higher the fluorescence intensity measured from the reaction product, the higher the ability of the compound to trap methylglyoxal. In addition, in this experimental example, when the fluorescence intensity was 10 X 10³ or more, it was determined that the compound had the ability to trap methylglyoxal.

**[Table 2]**

| Item | Fluorescence intensity on day 1 (X 10³) |
|---|---|
| PBS | 1.76±0.23 |
| MGO+PBS | 1.16±0.03 |
| Compound 8 | 21.74±0.50 |
| Compound 9 | 0.75±0.30 |
| Compound 10 | 223.01±3.74 |

As shown in Table 2 above and Fig. 1, it was confirmed that the compound of the present invention had an excellent ability to trap methylglyoxal.

### [Experimental Example 2]

### Analysis of methylglyoxal (MGO) trapping ability (2) : HPLC analysis

The objective of this experimental example was to confirm whether the compound of the present invention has the ability to trap methylglyoxal (MGO) by analyzing the concentration of free methylglyoxal (MGO) by high performance liquid chromatography (HPLC).

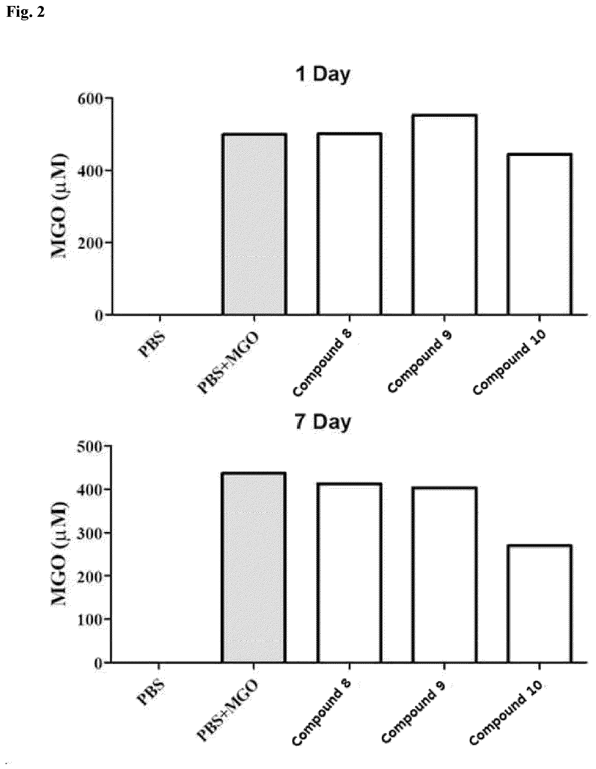

Specifically, a mixture was prepared by adding methylglyoxal (MGO) to phosphate buffered saline (PBS, Cat no. 10010023, pH 7.4). The compound of the above example was added to the mixture (MGO+PBS) at a concentration of 1:1 with methylglyoxal (MGO) and reacted, and then cultured at 37 °C for 1 day. On day 1 and day 7 after incubation, the concentration of free methylglyoxal (free MGO) that did not react with the compound of the above example was measured by HPLC, and the results are shown in Table 3 below and Fig. 2.

**[Table 3]**

| Item | MGO concentration on day 1 (µM) | MGO concentration on day 7 (µM) |
|---|---|---|
| PBS | 0 | 0 |
| MGO+PBS | 499.53 | 436.38 |
| Compound 8 | 501.09 | 412.65 |
| Compound 9 | 551.993 | 403.462 |
| Compound 10 | 444.11 | 269.28 |

As shown in Table 3 above and Fig. 2, it was confirmed that the compound of the present invention reduced the concentration of free methylglyoxal (free MGO) compared to the control group (N : PBS + MGO). In particular, when treated with Compound 10, the concentration of free methylglyoxal on day 1 after incubation was decreased to about 12% of the control group. In addition, on day 7 after incubation, when treated with Compound 10, it was decreased to about 38% of the control group. Therefore, it can be seen that the compound of the present invention has an excellent ability to trap methylglyoxal.

### [Experimental Example 3]

### Measurement of effect of protecting against cytotoxicity caused by treatment with MGO + _{L}-DOPA (1)

The objective of this experimental example was to confirm whether the compound of the present invention has the ability to protect against MGO + _{L}-DOPA when the cytotoxicity was induced by treatment with free MGO + _{L}-DOPA.

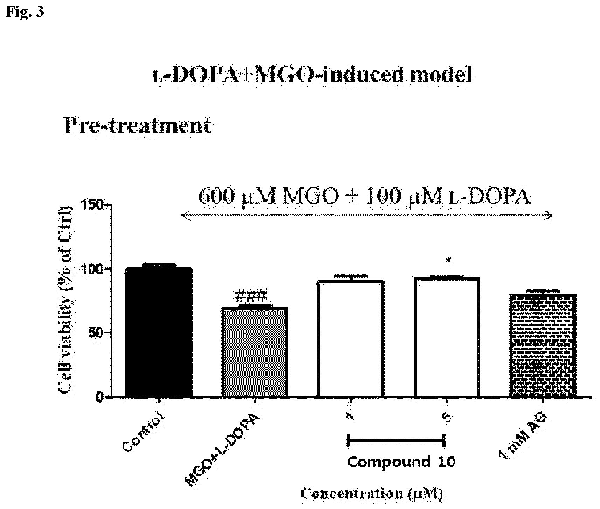

Specifically, SH-SY5Y cells were dispensed at 2 x 10⁴ cells/well in a 96 well plate. Each cell line was pre-treated with the compound of the above example at a concentration of 1 or 5 µM or aminoguanidine (AG) at a concentration of 1 mM for 1 hour, and then post-treated with 600 µM MGO + 100 µM _{L}-DOPA, and cultured for 24 hours. The medium was removed and then treated with 0.5 mg/ml 3-(4,5-dimethylthiazol-2-yl)-2,5-diphenyltetrazolium bromide (MTT) solution for 1 hour, and the reduced formazan was dissolved in 150 µl of DMSO, and the cell viability was measured by a microspectrophotometer at a wavelength of 570 nm. The cell viability of the untreated normal control group was set as 100%, and the cell viability when treated with each substance was evaluated, and the results are shown in Fig. 3 below.

As shown in Fig. 3, the cell viability in the group treated with MGO + _{L}-DOPA was significantly decreased compared to the normal control group, whereas the cell viability in the group treated with the compound of the present invention was increased in a concentration-dependent manner. Therefore, it can be seen that the compound of the present invention has an excellent effect of protecting against MGO + _{L}-DOPA.

### [Experimental Example 4]

### Measurement of effect of protecting against cytotoxicity caused by treatment with MGO + L-DOPA (2)

The objective of this experimental example was to confirm △FOSB expression factor involved in nerve cell death when the cytotoxicity was induced by treatment with free MGO + _{L}-DOPA.

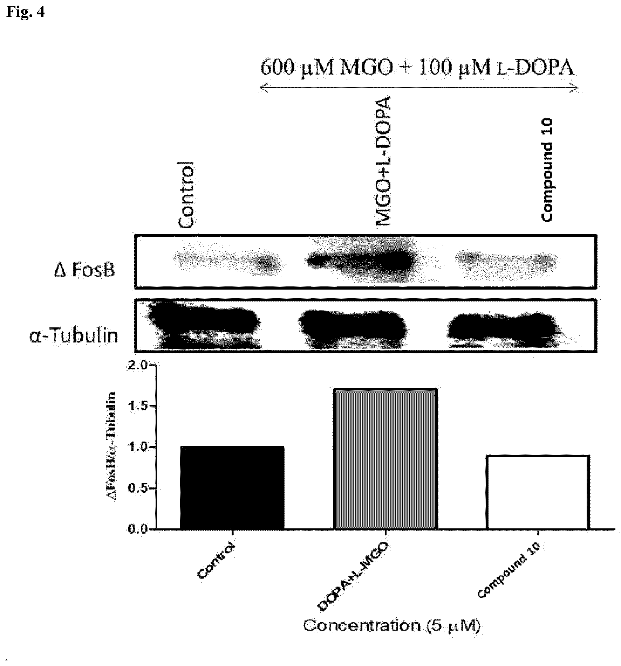

Specifically, SH-SY5Y cells were dispensed at 1 × 10⁶ cells/well in a 60 mm dish. Each cell line was pre-treated with the compound of the above example at concentration of 5 µM for 1 hour, and then post-treated with 600 µM MGO + 100 µM _{L}-DOPA, and cultured for 24 hours. After incubation, completely filled cells were washed with PBS and lysed using a lysis buffer (PRO-PREP^{™} Protein Extraction Solution, Intron Biotechnology, Seongnam, Korea). Thereafter, the protein content in the supernatant of the lysate was measured by the Bio-Rad Protein Assay (Bio-Rad, Califonia, USA) using bovine serum albumin (BSA) as a standard, and the total protein content in each sample was adjusted. Thereafter, for electrophoresis, the samples were loaded on a 10% SDS-PAGE gel so that the amount of protein in each sample was 30 µg, and transferred to a PVDF membrane. The membrane was blocked with 5% skim milk, and then antibodies to △FOSB and α-Tubulin (Cell Signaling Technologies, Massachusetts, USA) were each used to detect the membrane by the ChmiDoc XRS+ imaging system (Bio-Rad, CA, USA). The results are shown in Fig. 4 below.

As shown in Fig. 4, △FOSB expression was significantly increased in the group treated with MGO + _{L}-DOPA compared to the normal control group, whereas △FOSB expression was decreased in the group treated with the compound of the present invention. Therefore, it can be seen that the compound of the present invention has an excellent effect of protecting against MGO + _{L}-DOPA.

## Claims

1. A compound represented by Formula 1 below or a pharmaceutically acceptable salt thereof: 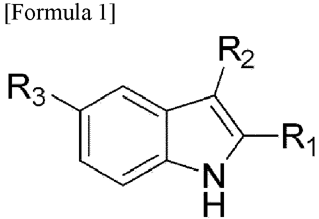 in the formula,
R₁ is hydrogen, C₁-C₆alkyl, or -C₁-C₆alkyl-N(Ra)(Rb),
R₂ is hydrogen, C₁-C₆alkyl, or -C₁-C₆alkyl-C(Rc)(N(Rd)(Re)), and
R₃ is hydrogen, -OH, -O-C(=O)C₁-C₆alkyl, -O-C(=O)C₂-C₆alkenyl, or -O-C(=O)Ci-C₆alkyl-C₃-C₇cycloalkyl, wherein
Ra and Rb are each independently hydrogen or C₁-C₆alkyl,
Rc is hydrogen, -C₁-C₆alkyl-OH, -C₁-C₆alkyl-O-C(=O)C₁-C₆alkyl or -C₁-C₆alkyl-O-C(=O)NH-C₁-C₆alkyl-NH-C₁-C₆alkyl-NH₂, and
Rd and Re are each independently hydrogen or -C(=O)C₁-C₆alkyl.

2. The compound represented by Formula 1 or pharmaceutically acceptable salt thereof according to claim 1, wherein Ri is hydrogen, C₁-C₆alkyl or -C₁-C₆alkyl-NH₂.

3. The compound represented by Formula 1 or pharmaceutically acceptable salt thereof according to claim 1, wherein R₁ is hydrogen or -C₁-C₆alkyl-NH₂.

4. The compound represented by Formula 1 or pharmaceutically acceptable salt thereof according to claim 1, wherein R₂ is hydrogen or -C₁-C₆alkyl-C(Rc)(N(Rd)(Re)), wherein
Rc is hydrogen, -C₁-C₆alkyl-OH, -C₁-C₆alkyl-O-C(=O)C₁-C₆alkyl or -C₁-C₆alkyl-O-C(=O)NH-C₁-C₆alkyl-NH-C₁-C₆alkyl-NH₂, and
Rd and Re are each independently hydrogen or -C(=O)C₁-C₆alkyl.

5. The compound represented by Formula 1 or pharmaceutically acceptable salt thereof according to claim 1, wherein R₂ is hydrogen,

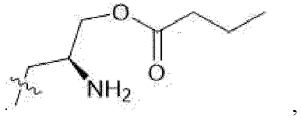

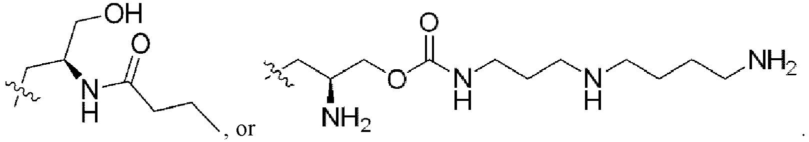

6. The compound represented by Formula 1 or pharmaceutically acceptable salt thereof according to claim 1, wherein R₃ is hydrogen, -OH,

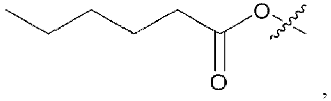

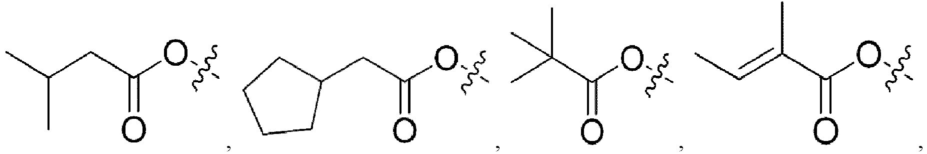

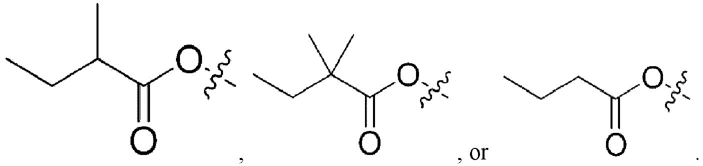

7. A compound selected from the group consisting of the following compounds or a pharmaceutically acceptable salt thereof:
| Compound | Structural formula |
|---|---|
| 1 | 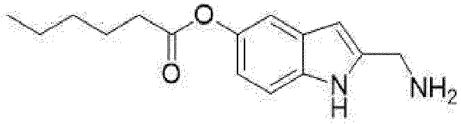 |
| 2 | 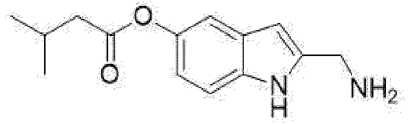 |
| 3 | 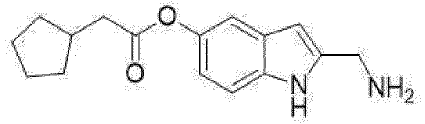 |
| 4 | 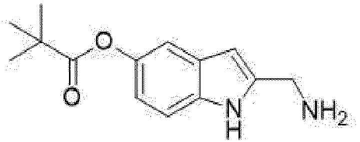 |
| 5 | 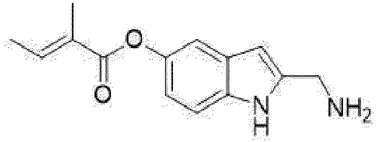 |
| 6 | 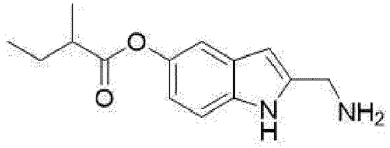 |
| 7 | 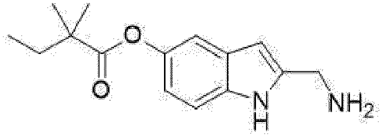 |
| 8 | 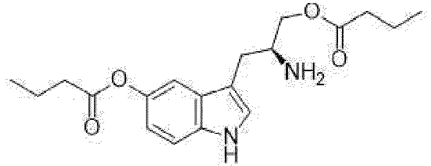 |
| 9 | 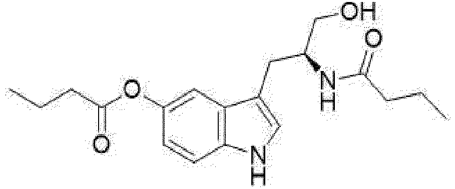 |
| 10 | 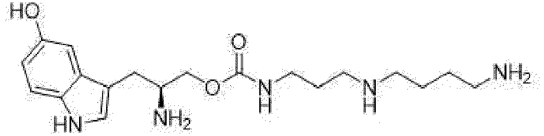 |

8. A pharmaceutical composition for preventing or treating diseases related to advanced glycation end products, comprising the compound or pharmaceutically acceptable salt thereof according to any one of claims 1 to 7.

9. The pharmaceutical composition according to claim 8, wherein the disease related to advanced glycation end products is selected from the group consisting of aging, diabetes mellitus, diabetic complications, hyperlipidemia, hyperglycemia, cardiovascular disease, degenerative brain disease, autism spectrum disorder, arteriosclerosis, non-alcoholic fatty liver disease, non-alcoholic steatohepatitis, skin fibrosis, pulmonary fibrosis, renal fibrosis, and cardiac fibrosis.

10. The pharmaceutical composition according to claim 8, wherein the disease related to advanced glycation end products is diabetes mellitus or diabetic complications.

11. The pharmaceutical composition according to claim 10, wherein the diabetes mellitus is type 2 diabetes mellitus.

12. The pharmaceutical composition according to claim 10, wherein the diabetic complications are selected from the group consisting of diabetic nephropathy, diabetic retinopathy, diabetic cataract, diabetic neuropathy, diabetic foot ulcer, diabetic cardiovascular disease, diabetic arteriosclerosis, diabetic osteoporosis, diabetic sarcopenia, and obesity.

13. The pharmaceutical composition according to claim 9, wherein the degenerative brain disease is selected from the group consisting of Alzheimer's disease, Parkinson's disease, Huntington's disease, Pick's disease, Creutzfeldt-Jakob disease, Lou Gehrig's disease, spinocerebellar degeneration, Friedreich's ataxia, spinocerebellar ataxia, Machado-Joseph's disease, dystonia, progressive supranuclear palsy, cognitive dysfunction, senile dementia, Lewy body dementia, frontotemporal dementia, vascular dementia, alcoholic dementia, presenile dementia, temporal lobe epilepsy, and stroke.

14. A food composition for preventing or improving diseases related to advanced glycation end products, comprising the compound or pharmaceutically acceptable salt thereof according to any one of claims 1 to 7.

15. The food composition according to claim 14, wherein the disease related to advanced glycation end products is selected from the group consisting of aging, diabetes mellitus, diabetic complications, hyperlipidemia, hyperglycemia, cardiovascular disease, degenerative brain disease, autism spectrum disorder, arteriosclerosis, non-alcoholic fatty liver disease, non-alcoholic steatohepatitis, skin fibrosis, pulmonary fibrosis, renal fibrosis, and cardiac fibrosis.

16. The food composition for preventing or improving diseases related to advanced glycation end products according to claim 14, wherein the disease related to advanced glycation end products is diabetes mellitus or diabetic complications.

17. The food composition according to claim 16, wherein the diabetes mellitus is type 2 diabetes mellitus.

18. The food composition for preventing or improving diseases related to advanced glycation end products according to claim 16, wherein the diabetic complications are selected from the group consisting of diabetic nephropathy, diabetic retinopathy, diabetic cataract, diabetic neuropathy, diabetic foot ulcer, diabetic cardiovascular disease, diabetic arteriosclerosis, diabetic osteoporosis, diabetic sarcopenia, and obesity.

19. The food composition for preventing or improving diseases related to advanced glycation end products according to claim 15, wherein the degenerative brain disease is selected from the group consisting of Alzheimer's disease, Parkinson's disease, Huntington's disease, Pick's disease, Creutzfeldt-Jakob disease, Lou Gehrig's disease, spinocerebellar degeneration, Friedreich's ataxia, spinocerebellar ataxia, Machado-Joseph's disease, dystonia, progressive supranuclear palsy, cognitive dysfunction, senile dementia, Lewy body dementia, frontotemporal dementia, vascular dementia, alcoholic dementia, presenile dementia, temporal lobe epilepsy, and stroke.

20. An animal feed composition for preventing or improving diseases related to advanced glycation end products, comprising the compound or pharmaceutically acceptable salt thereof according to any one of claims 1 to 7.
